# EUROPEAN PATENT APPLICATION

(11) **EP 4 541 811 A1**
(43) Date of publication of application: **23.04.2025**
(21) Application number: 23823090.8
(22) Date of filing: 12.06.2023
(51) Int. Cl.: C07K 14/725, C12N 15/12, C12N 15/63, C12N 5/10, A61K 39/00, A61P 35/00

(54) **T CELL RECEPTOR TARGETING KRAS G12V MUTANT POLYPEPTIDE, AND USE THEREOF**

(30) Priority: 14.06.2022 CN 202210685197
(71) Applicant: Immuxell Biotech Ltd., Shanghai 201315 (CN)
(72) Inventor: ZOU, Xuemei, Shanghai 201315 (CN); ZHANG, Xiaohui, Shanghai 201315 (CN); GAN, Chi, Shanghai 201315 (CN); WEN, Maorong, Shanghai 201315 (CN); YU, Guangjie, Shanghai 201315 (CN)
(74) Representative: Viering, Jentschura & Partner mbB Patent- und Rechtsanwälte
(86) International application number: PCT/CN2023/099745
(87) International publication number: WO 2023/241522

(57) **Abstract**

Provided are a T cell receptor targeting a KRAS G12V mutant polypeptide and a use thereof. The T cell receptor molecule specifically targets a KRAS G12V mutation; a CDR3 sequence of an α chain variable domain thereof contains CAVRDIEGAGNNRKLIW (SEQ ID NO: 1) or a mutant of SEQ ID NO: 1, and/or a CDR3 sequence of a β chain variable domain contains CASSEGQYSYEQYF (SEQ ID NO: 2) or a mutant of SEQ ID NO: 2. Further provided is a nucleic acid molecule encoding the T cell receptor molecule, a dual-targeting protein molecule and a multivalent complex comprising the T cell receptor molecule, a nucleic acid construct, a cell expressing the T cell receptor molecule, etc.

## Description

### TECHNICAL FIELD

The present invention relates to T cell receptor sequences targeting KRAS G12V mutant polypeptides and nucleotide sequences encoding thereof. The present invention also relates to a TCR-T cell therapy method developed based on the T cell receptor, as well as dual-targeted anti-tumor protein medicine developed based on the T cell receptor.

### BACKGROUND

Adoptive cellular immunotherapy is a newly developed cutting-edge technology that has achieved unprecedented success in the treatment of hematological tumors. However, adoptive cellular immunotherapy technology has many difficulties in the treatment of solid tumors. Finding suitable targets and developing receptor molecules that specifically bind to these targets can break new ground in the treatment of solid tumors. The KRAS gene has come into the researchers' view. KRAS is one of the main members of the Ras gene family (including NRAS, HRAS and KRAS). It is a murine sarcoma virus oncogene, located on chromosome 12, about 35kb long, encoding KRAS protein. When KRAS mutates, it continuously binds to GTP and exhibits tyrosine kinase activity to activate downstream signaling pathways, thereby causing uncontrolled cell proliferation and leading to the occurrence of tumors. Studies have found that KRAS mutations exist in approximately 30% of tumors, including 90% of pancreatic cancers, 50% of colon cancers and 25% of lung cancers.

KRAS mutations often occur at positions such as glycine at position 12, glycine at position 13, and glutamine at position 61. The proportion of mutations in glycine at position 12 and glycine at position 13 is as high as 97%, which mainly are G12C, G12D, G12V, G12R and G13D mutations. KRAS G12V mutations are expressed in approximately 30% of pancreatic cancer and 10% of colorectal cancer or non-small cell lung cancer. Moreover, RAS family members also share G12V hotspot mutations in different cancer types (e.g., NRAS in melanoma). Although KRAS mutations are found in many tumors, KRAS has been regarded as an undruggable target for many years due to the lack of a pocket on its surface to bind small molecule inhibitors. Currently, there is only one KRAS G12C inhibitor, AMG 510, approved for marketing for the treatment of patients with advanced non-small cell lung cancer (NSCLC) carrying KRAS G12C mutations who have previously received systemic therapy. However, KRAS G12C is rarely found in other cancers (such as pancreatic cancer, colon cancer, etc.). For other high-abundant KRAS mutation types, such as G12D and G12V, it is also urgent to find new treatments.

Addressing high-frequency KRAS mutations through adoptive cellular immunotherapy is currently a breakthrough point in research. One type of adoptive cellular immunotherapy, called T-cell receptor-engineered T-cell therapy (TCR-T), has obvious advantages. TCR-T therapy utilizes the tumor-killing properties of T cells, transfers tumor-specific TCR genes into T cells, and mediates the specific recognition of tumor antigens by T cells through the receptors expressed by the T cells, ultimately realizing the recognition and killing effects on tumors. TCR-T cell therapy has demonstrated good safety and effectiveness in clinical trials globally for the treatment of refractory relapsed melanoma, synovial sarcoma, multiple myeloma and lung cancer. Finding TCR receptors with high specificity and strong affinity is the key and technical fortress of TCR-T technology.

### SUMMARY

A first aspect of the present invention provides a T cell receptor (TCR) molecule that specifically targets the KRAS G12V mutation, wherein a CDR3 sequence of an α chain variable region thereof contains CAVRDIEGAGNNRKLIW (SEQ ID NO: 1) or a mutant of SEQ ID NO: 1, and/or a CDR3 sequence of a β chain variable region contains CASSEGQYSYEQYF (SEQ ID NO: 2) or a mutant of SEQ ID NO: 2.

A second aspect of the present invention provides a multivalent TCR complex, which comprises two or more TCR molecules according to any embodiment of the present invention.

The third aspect of the present invention provides a dual-targeting protein molecule that can simultaneously bind to tumor cells and immune cells, the dual-targeting protein molecule includes the TCR molecule targeting the KRAS G12V mutation on the surface of tumor cells according to any embodiment herein and a single-chain antibody (scFv) for recruiting and redirecting immune cells to the periphery of tumor cells, wherein the signal peptide and transmembrane domain in the α chain variable region and β chain variable region of the TCR molecule are deleted.

The fourth aspect of the present invention provides a nucleic acid molecule comprising a nucleic acid sequence encoding a TCR molecule or a dual-targeting protein molecule according to any embodiment of the present invention or a complementary sequence thereof.

The fifth aspect of the present invention provides a nucleic acid construct comprising a nucleic acid molecule as described in any embodiment of the present invention.

The sixth aspect of the present invention provides an isolated cell, the cell:
(1) contains a nucleic acid construct described in any embodiment of the present invention or contains a nucleic acid molecule described in any embodiment of the present invention integrated into the chromosome, and/or
(2) expresses a TCR molecule as described in any embodiment of the present invention or a dual-targeting protein molecule as described in any embodiment of the present invention.

Preferably, the cell is immune effector cell, preferably T cell, NK cell, and TIL cell.

The seventh aspect of the present invention provides a pharmaceutical composition comprising a pharmaceutically acceptable carrier and a TCR molecule, a TCR complex, a dual-targeting protein molecule, a nucleic acid molecule, a recombinant expression vector or a cell as described in any embodiment of the present invention.

The eighth aspect of the present invention provides the use of TCR molecule, TCR complex, dual-targeting protein molecule, nucleic acid molecule, recombinant expression vector or cell described in any embodiment of the present invention in the manufacture of a medicament for the treatment or prevention of diseases related to the KRAS G12V mutant antigen in patients.

The ninth aspect of the present invention provides a method for treating and/or preventing a disease associated with the KRAS G12V mutant antigen of a patient, which comprises the step of adoptive transfer to the patient of a T cell containing a vector of the present invention or a T cell containing a nucleic acid molecule of the present invention integrated in chromosome, and/or a T cell expressing a TCR molecule described in any embodiment of the present invention, or comprises the step of administering the patient a dual-targeting protein molecule described in any embodiment of the present invention or a pharmaceutical composition containing the dual-targeting protein molecule.

The above aspects of the invention are described in detail as follows.

### Description of Drawings

Figure 1: Schematic diagram of pMSGV1_051 TCR vector.
Figure 2: Cell positivity rate after transfection with TCR. The T cells transfected with TCR were stained with mTCR-PE and MHC tetramers, and the cell ratio of PE channel and APC channel was detected by flow cytometry, respectively.
Figure 3: The assay of KRAS wild-type (SEQ ID NO: 13, SEQ ID NO: 14) and G12V peptides (SEQ ID NO: 15, SEQ ID NO: 16) activating TCR-T. TCR-T cells were incubated with KRAS wild-type and G12V polypeptides at different concentrations for 12 hours. Flow cytometry was then used to detect the expression of 4-1BB and mTCR on the surface of TCR-T cells.
Figure 4: The KRAS G12V mutant peptide VVVGAVGVGK activates 051 TCR-T with EC₅₀=0.9 ng/ml.
Figure 5: TCR-T specifically recognizes tumor cells with both HLA-A*11:01 and KRAS G12V mutation features. TCR-T cells were co-cultured with different tumor cells for 16 hours, and the expression of 4-1BB on the surface of TCR-T cells was measured to characterize T cell activation. PANC1 was HLA-A*11:01 and KRAS was mutated to G12D. SW620 has a KRAS mutation of G12V, but not of the HLA-A*11:01 genotype. CD3 antibody stimulation was the positive control group, and N.C. was the unstimulated negative control group.
Figure 6: The anti-tumor effect of TCR-T cell therapy is observed in a mouse tumor model.
Figure 7: Dual-targeting protein molecule TCR-ICE design. A, TCR-ICE protein molecular domain; B, Protein structure model showing TCR-ICE protein molecule.
Figure 8: Purification of TCR-ICE protein molecule was identified by SDS-PAGE. A, Identification of TCR-ICE inclusion bodies by SDS-PAGE; B. Identification of TCR-ICE purification by SDS-PAGE.
Figure 9: Detection of TCR-ICE-activated T cells by flow cytometry analyzer. A. gating strategy for detecting the expression of 4-1BB and OX40 on the surface of peripheral blood lymphocytes. B. Expression of 4-1BB and OX40 on the surface of T cells after treatment with different concentrations of TCR-ICE.
Figure 10: TCR-ICE activates T cells to secrete interferon-γ. A, TCR-ICE and target cell K562-TMG-A11 activate T cells to secrete interferon-γ. B, TCR-ICE and target cell CFPAC1-A11 activate T cells to secrete interferon-γ. The CD3/CD28/CD2 group serves as a positive control group, wherein CD3/CD28/CD2 antibodies are added to stimulate T cells. The No TCR-ICE group is the group of mixed culture of effector cells and target cells without adding TCR-ICE and serves as a negative control group.
Figure 11: TCR-ICE inhibits the growth of pancreatic cancer tumors in vivo mice.

### DETAILED DESCRIPTION

The present invention discovers a TCR molecule that specifically targets the KRAS G12V mutation antigen (especially specifically targets the KRAS G12V mutation antigen shown in SEQ ID NO: 16), and the TCR molecule can specifically bind to KRAS G12V/HLA-A*11:01 complex on the surface of tumor cells, but normal non-cancer cells are not recognized because they express unmutated wild-type KRAS protein. Therefore, the TCR molecule of the present invention has strong specificity, so that the toxic side effects of the T cells expressing the TCR molecule in a medicine are reduced, and the normal non-cancer cells are not destroyed, and it has a wide range of applications in the treatment of tumors (such as pancreatic cancer, colorectal cancer, lung cancer, endometrial cancer, ovarian cancer and prostate cancer, etc., especially pancreatic cancer), and thus the present invention is completed.

The present invention will be described in detail below. It should be understood that within the scope of the present invention, the above-mentioned technical features of the present invention and the technical features specifically described below (such as embodiments) can be combined with each other to form a preferred technical solution.

### Definition of Terms

Herein, TCR has a well-known meaning in the art, it is a glycoprotein on the surface of the cell membrane in the form of a heterodimer of the α/β chain or the γ chain/δ chain, which is a characteristic marker on the surface of all T cells. T cell receptors form a T cell receptor complex together with constant CD3 molecules. TCR is a receptor for intracellular antigen peptides presented by the major histocompatibility complex (MHC). The TCR of most T cells is composed of a dimer composed of α and β peptide chains, and the TCR of a few T cells is composed of γ and δ peptide chains. Each subunit contains two extracellular regions: a variable region and a constant region. The constant region is close to the cell membrane and connected to the transmembrane domain, while the variable region is responsible for recognizing the peptide/MHC complex. The variable regions contain three highly variable complementarity determining regions (CDRs), namely CDR1, CDR2 and CDR3. The most important CDR3 is responsible for directly binding to peptides presented by MHC. CDR1 of the α subunit and β subunit act on the N-terminus and C-terminus of the polypeptide respectively. CDR2 is thought to be involved in recognizing MHC. The β subunit also has an additional CDR4, which is usually not involved in the recognition of peptide/MHC complexes, but is related to the role of superantigens.

Herein, the major histocompatibility complex (MHC) is a gene family present in most vertebrate genomes, which is an antigen-presenting and T cell activation molecule. The human MHC glycoprotein is also known as human leukocyte antigen (HLA). MHC molecules include class I and class II MHC molecules. MHC molecules can present degraded fragments of intracellular proteins, for example, after the cell is infected by a virus, the peptide fragments of the corresponding viral outer membrane can be presented to the cell surface through MHC molecules, so that CD8+ cytotoxic T cells can identify and specifically kill the cells infected by the virus.

Herein, amino acid residues are described by the following abbreviations: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), valine (Val or V). In addition, in the present specification, the amino acid sequence of a peptide is described in a conventional manner with the amino terminus (hereinafter referred to as the N terminus) located on the left and the carboxyl terminus (hereinafter referred to as the C terminus) located on the right.

The term "T cell receptor targeting KRAS G12V mutation (KRAS^{G12V}-reactive T Cell Receptor)" is defined herein as a TCR molecule that is capable of binding to a KRAS G12V mutant polypeptide/MHC complex, thereby inducing T cell toxicity. In particular, the KRAS G12V mutant polypeptide includes but is not limited to the amino acid sequence shown in SEQ ID NO: 16, and the MHC is HLA-A*11:01.

The term "exogenous T cell receptor" (exogenous TCR) is defined herein as a recombinant TCR expressed in a cell by the introduction of exogenous coding sequences. The TCR targeting KRAS G12V mutation provided in this article is an "exogenous T cell receptor" for human T cells, and it can be expressed in human T cells. The human T cells naturally expressing endogenous TCR are insufficient to induce cells or responsive cells to react to the binding of TCR ligands.

Herein, TCR-T cell therapy involves introducing exogenous TCR genes into ordinary T cells, enabling the modified T cells to express TCRs that can effectively recognize tumor cells and thereby guiding T cells to kill tumor cells. This therapy usually includes the step of administering to the patient T cells that have been modified to express exogenous TCR genes. T cells usually come from the patients themselves. Typically, T cells are obtained from the patient, modified in vitro to express exogenous TCR genes (such as the TCR genes described in any of the embodiments herein), and then transfused back to the patient.

Herein, the dual-targeting protein molecule is a class of artificial protein molecules designed based on the BiTE (Bi-specific T-cell engagers) strategy, and one end of the protein is a high-affinity T cell receptor (TCR), which can target the KRAS G12V mutation on the surface of tumor cells; the other end is a single-chain antibody (scFv), which is used to recruit and redirect immune cells to the periphery of tumor cells. An exemplary single-chain antibody can be an anti-CD3 single-chain antibody, and an exemplary immune cell can be a T cell. TCR first recognizes and binds to the polypeptide/MHC on the surface of tumor cells. Then, the anti-CD3 antibody fragment recruits and redirects immune cells to the periphery of tumor cells. In this way, the dual-targeting protein molecule builds a bridge between cancer cells and immune cells, forms an immune synapse, activates immune cells and releases soluble particles, leading to cancer cell death.

Herein, sequence identity can be determined by methods known in the art, for example, BLASTP can be used to determine the sequence identity of two aligned amino acid sequences. "Conservative substitutions" are known in the art as substitutions in which one or more amino acid residues are replaced by one or more amino acid residues having a side chain R group with similar chemical properties (e.g., charge or hydrophobicity). Generally, conservative amino acid substitutions do not substantially change the functional properties of the protein. Examples of groups of amino acids with side chains of similar chemical properties include: 1. aliphatic side chains: glycine, alanine, valine, leucine, and isoleucine; 2. aliphatic hydroxyl side chains: serine and threonine; 3. amide-containing side chains: asparagine and glutamine; 4. aromatic side chains: phenylalanine, tyrosine, and tryptophan; 5. basic side chains: lysine, arginine, and histidine; 6. acidic side chains: aspartic acid and glutamic acid; and 7. sulfur-containing side chains: cysteine and methionine. Amino acids can be classified according to the polarity of their side chain groups as follows: 1. Non-polar amino acids (hydrophobic amino acids), including alanine, valine, leucine, isoleucine, proline, phenylalanine, tryptophan and methionine; 2. Polar amino acids (hydrophilic amino acids), including polar uncharged (neutral amino acids) such as glycine, serine, threonine, cysteine, tyrosine, asparagine, glutamine, selenocysteine and pyrrolysine, and polar positively charged amino acids (basic amino acids), including lysine, arginine and histidine; 3. Polar negatively charged amino acids (acidic amino acids), including aspartic acid and glutamic acid.

Herein, immune cells refer to cells involved in or associated with immune responses, generally including lymphocytes, dendritic cells, monocytes/macrophages, granulocytes, mast cells, etc. Exemplary immune cells include T cells, natural killer cells (NK) and tumor infiltrating lymphocytes (TIL), etc., as well as their derived immune cells, such as stem cells such as hematopoietic stem cells (HSC) and induced pluripotent stem cells (iPS), etc.

### T cell receptor (TCR)

The characteristics of the TCR molecule targeting KRAS G12V mutation of the present invention include that the CDR3 sequence of the variable region of its α chain contains CAVRDIEGAGNNRKLIW (SEQ ID NO: 1) or a mutant of SEQ ID NO: 1, and/or the CDR3 sequence of the variable region of the β chain contains CASSEGQYSYEQYF (SEQ ID NO: 2) or a mutant of SEQ ID NO: 2. Preferably, compared with SEQ ID NO: 1, the mutant of SEQ ID NO: 1 has 1-5 (such as 1, 2 or 3) amino acid mutations, or has at least 80%, preferably at least 85%, preferably at least 90%, preferably at least 95%, preferably at least 98% sequence identity, and retains the binding activity of SEQ ID NO: 1 as the CDR3 of the variable region of the TCR α chain. Preferably, compared with SEQ ID NO: 2, the mutant of SEQ ID NO: 2 has 1-5 (such as 1, 2 or 3) amino acid mutations, or has at least 80%, preferably at least 85%, preferably at least 90%, preferably at least 95%, preferably at least 98% sequence identity, and retains the binding activity of SEQ ID NO: 2 as the CDR3 of the variable region of the TCR β chain. The mutations contained in the mutants of SEQ ID NO: 1 and 2 respectively can be selected from one or more of insertion, deletion and substitution. Preferably, the mutation is a conservative mutation, such as a conservative substitution.

In some embodiments, the CDR1 sequence of the variable region of the TCR α chain of the present invention contains SVSGNP (SEQ ID NO: 3) or its mutant, and the CDR2 sequence contains YITGDN (SEQ ID NO: 4) or its mutant. In some embodiments, the CDR1 sequence of the variable region of the TCR β chain of the present invention contains SNHLY (SEQ ID NO: 5) or its mutant, and the CDR2 sequence contains FYNNEI (SEQ ID NO: 6) or its mutant. The mutants of SEQ ID NO: 3, 4, 5 and 6 respectively can have 1, 2 or 3 amino acid mutations compared to themselves, including but not limited to one or more of insertion, deletion and substitution, and this mutation does not affect the biological functions and activities of these CDR sequences in the TCR molecule. Preferred mutations are conservative mutations, such as conservative substitutions.

In some embodiments, the CDR1 sequence of the variable region of the α chain of the TCR molecule of the present invention is SVSGNP (SEQ ID NO: 3), the CDR2 sequence is YITGDN (SEQ ID NO: 4), and the CDR3 sequence is CAVRDIEGAGNNRKLIW (SEQ ID NO: 1); and/or, the CDR1 sequence of the variable region of the β chain is SNHLY (SEQ ID NO: 5), the CDR2 sequence is FYNNEI (SEQ ID NO: 6), and the CDR3 sequence is CASSEGQYSYEQYF (SEQ ID NO: 2).

The amino acid sequences of the CDR regions of the TCR molecule of the present invention can be embedded into any suitable framework structure to prepare chimeric TCRs. As long as the framework structure is compatible with the CDR regions of the TCR of the present invention, those skilled in the art can design or synthesize TCR molecules with corresponding functions according to the CDR regions disclosed in the present invention. Therefore, the TCR molecule of the present invention refers to a TCR molecule containing the above α and/or β chain CDR region sequences and any suitable framework structure that adopts the CDR region sequence of the present invention.

In some embodiments, the variable region of the α chain of the TCR molecule of the present invention contains or consists of the amino acid sequence shown in SEQ ID NO: 7, or contains or consists of an amino acid sequence with one or more mutations compared to the amino acid sequence shown in SEQ ID NO: 7, or contains or consists of an amino acid sequence with at least 80%, at least 85%, at least 90%, preferably at least 95%, more preferably at least 98% sequence identity compared to the amino acid sequence shown in SEQ ID NO: 7. The number of mutated amino acid residues can be, for example, 1-15, such as 1-10 or 1-5 mutations; the mutations can be selected from one or more of insertion, deletion and substitution. Mutations can occur in any domain of SEQ ID NO: 7, including in its CDR and/or FR regions. In some embodiments, the mutation does not occur in the sequences of CDR1, CDR2 and CDR3 contained in SEQ ID NO: 7. In some embodiments, the mutation occurs, for example, in the FR region of SEQ ID NO: 7. Preferably, the mutation is a conservative mutation, such as a conservative substitution.

In some embodiments, the variable region of the β chain of the TCR molecule of the present invention contains or consists of the amino acid sequence shown in SEQ ID NO: 8, or contains or consists of an amino acid sequence with one or more mutations compared to the amino acid sequence shown in SEQ ID NO: 8, or contains or consists of an amino acid sequence with at least 80%, at least 85%, at least 90%, preferably at least 95%, more preferably at least 98% sequence identity compared to the amino acid sequence shown in SEQ ID NO: 8. The number of mutated amino acid residues can be, for example, 1-15, such as 1-10 or 1-5 mutations; the mutations can be selected from one or more of insertion, deletion and substitution. Mutations can occur in any domain of SEQ ID NO: 8, including in its CDR and/or FR regions. In some embodiments, the mutation does not occur in the sequences of CDR1, CDR2 and CDR3 contained in SEQ ID NO: 8. In some embodiments, the mutation occurs, for example, in the FR region of SEQ ID NO: 8. Preferably, the mutation is a conservative mutation, such as a conservative substitution.

It should be understood that the mutants of the TCR molecule of the present invention (that is, containing the mutant of the variable region of the α chain and/or the mutant of the variable region of the β chain as described above) still retain the TCR molecule containing SEQ ID NO: 1 and SEQ ID NO: 2 (especially the TCR molecule containing SEQ ID NO: 7 and 8) depends on HLA-A*11:01 to specifically bind to the biological activity of KRAS G12V mutation (especially the polypeptide shown in SEQ ID NO: 16).

In some embodiments, the TCR molecule of the present invention is a dimer composed of α and β chains. The α chain contains a variable region and a constant region. The CDR1 sequence of the variable region of the α chain is SVSGNP (SEQ ID NO: 3), and the CDR2 sequence is YITGDN (SEQ ID NO: 4), the CDR3 sequence is CAVRDIEGAGNNRKLIW (SEQ ID NO: 1), the CDR1 sequence of the variable region of the β chain is SNHLY (SEQ ID NO: 5), the CDR2 sequence is FYNNEI (SEQ ID NO: 6), and the CDR3 sequence is CASSEGQYSYEQYF (SEQ ID NO: 2). In some embodiments, the variable region of the α chain contains the amino acid sequence shown in SEQ ID NO: 7 or the mutant sequence described above, and the variable region of the β chain contains the amino acid sequence shown in SEQ ID NO: 8 or the mutant sequence described above. To further stabilize the formation of the TCR α and β chain dimer, a disulfide bond can be introduced between the α chain and the β chain to form a dimer.

In some embodiments, the constant region of the TCR molecule of the present invention is a human constant region. The amino acid sequence of the human constant region can be obtained from publicly available databases.

Studies have shown that replacing the constant region of human TCR with the constant region of mouse TCR can effectively avoid the rearrangement of exogenous T cell receptors in the human body and its own TCR, resulting in off-target or even binding to wrong targets. Therefore, in some embodiments, the TCR molecule of the present invention contains the mouse α constant region and β constant region. An exemplary amino acid sequence of the mouse α constant region is shown as SEQ ID NO: 9, and the amino acid sequence of the β constant region is shown as SEQ ID NO: 10.

In some embodiments, the TCR molecule of the present invention is a TCR molecule from which the signal peptide sequence and transmembrane sequence of the variable region have been removed. An exemplary amino acid sequence of such a variable region of the α chain is shown as SEQ ID NO: 19; an exemplary amino acid sequence of such a variable region of the β chain is shown as SEQ ID NO: 20. This TCR molecule may contain the α constant region and β constant region of a human TCR molecule. The present invention also includes mutants of SEQ ID NO: 19 and 20, that is, compared with SEQ ID NO: 19 and 20, their respective mutants have one or more (for example, 1-15, such as 1-10 or 1-5) mutations, or have at least 80%, at least 85%, at least 90%, at least 95%, at least 98% sequence identity. The mutations of the mutant can occur in any one or more CDRs (such as any one or more CDR mutants as described above), or occur in the FR region. The mutations can be selected from one or more of insertion, deletion and substitution. Preferably, the mutation is a conservative mutation, such as a conservative substitution.

In some embodiments, the TCR of the present invention is provided in the form of a multivalent complex. The multivalent TCR complex of the present invention contains a polymer formed by combining two, three, four or more TCR molecules of the present invention.

### Nucleic acid molecules

The present invention provides nucleic acid molecules encoding the variable region of the α chain, the variable region of the β chain, the α chain, the β chain and the TCR molecule as described in any of the embodiments herein.

The nucleotide sequence of the nucleic acid molecule of the present invention can be single-stranded or double-stranded. The nucleic acid molecule can be RNA or DNA and may or may not contain introns. An exemplary sequence of a nucleic acid molecule encoding the variable region of the α chain of the present invention is shown as SEQ ID NO: 11. An exemplary polynucleotide sequence encoding the variable region of the β chain of the present invention is shown as SEQ ID NO: 12. In some embodiments, the variable region of the α chain and the variable region of the β chain of the present invention are modified to delete the signal peptide sequence and transmembrane sequence, and their exemplary coding sequences are shown as SEQ ID NO: 23 and SEQ ID NO: 24, respectively.

In some embodiments, the TCR molecule of the present invention comprises the human variable region and the mouse constant region, in the mouse constant region, the nucleic acid coding sequence of the α constant region can be as shown in SEQ ID NO: 17, and the nucleic acid coding sequence of the β constant region can be as shown in SEQ ID NO: 18.

It should be understood that due to the degeneracy of the genetic code, different nucleotide sequences can encode the same polypeptide. Therefore, the nucleic acid sequences encoding the variable region of the α chain, the variable region of the β chain, the α chain, the β chain and the TCR molecule of the present invention can be the same as the nucleic acid sequences shown in the present invention or degenerate variants. To illustrate with one of the examples in the present invention, a "degenerate variant" refers to a nucleic acid sequence that encodes a protein sequence having SEQ ID NO: 7 or 8 but is different from the sequence of SEQ ID NO: 7 or 8.

To efficiently express TCR in T cells, the nucleotide sequence of the present invention can be optimized by codon optimization methods. Different cells are different in the utilization of specific codons. According to the type of cell, the codons in the sequence can be changed to increase the expression level. The optimized codon for mammalian cells and many other organisms are known to those skilled in the art.

The full-length sequence or fragment of the nucleic acid molecule of the present invention can usually be obtained by, but not limited to, PCR amplification method, recombination method or artificial synthesis method. At present, it is possible to completely obtain the DNA sequence encoding the TCR (or its fragment, or its derivative) of the present invention through chemical synthesis. Then this DNA sequence can be introduced into various existing DNA molecules (such as vectors), mRNA or cells known in the art. DNA or mRNA can be the coding strand or the noncoding strand.

### Nucleic acid constructs

The present invention also includes nucleic acid constructs containing a nucleic acid molecule as described in any embodiment herein.

The nucleic acid construct herein can be an expression cassette which contains a promoter sequence, the nucleic acid molecule as described in any of the embodiments herein, and a polyA tail, which are operably linked. The expression cassette can also contain other regulatory elements operably linked to the above elements, such as enhancers and the like.

In some embodiments, the nucleic acid construct is a vector. Herein, vectors include but are not limited to expression vectors and cloning vectors. An expression vector refers to a vector used for expressing the TCR of the present invention in vivo or in vitro, and a cloning vector refers to a vector used in the preparation of the nucleic acid molecule of the present invention. Expression vectors usually include expression regulatory elements. Expression regulatory elements are well known in the art and include, but are not limited to, promoters and enhancers. In some embodiments, the vector contains the expression cassette.

Herein, expression vectors can be related vectors based on viral delivery systems, including but not limited to adenovirus vectors, adeno-associated virus (AAV) vectors, herpes virus vectors, retrovirus vectors, lentivirus vectors and baculovirus vectors; or non-viral delivery system vectors, including but not limited to expression vectors based on transposons, vectors based on gene editing methods, etc. Ideally, a suitable vector can transfer the TCR nucleic acid of the present invention into cells, such as T cells, so that the cells express TCR specific for KRAS G12V mutant antigen.

### Dual-targeting protein molecules

In some embodiments, the present invention provides a dual-targeting protein molecule that can simultaneously bind to tumor cells and immune cells (especially T cells). The dual-targeting protein molecule includes a TCR molecule that can target the KRAS G12V mutation on the surface of tumor cells as described in any embodiment of this article and a single-chain antibody (scFv) used to recruit and redirect immune cells to the periphery of the tumor cells. The TCR molecule may include an α chain variable region and an α chain constant region and a β chain variable region and a β chain constant region of the TCR molecule. The variable region and the constant region may be directly connected, or may be connected by a flexible peptide chain. Usually, the signal peptide and transmembrane domain in the α chain variable region and β chain variable region are deleted; an exemplary amino acid sequence of such α chain variable region is shown in SEQ ID NO: 19; exemplary; the amino acid sequence of this type of β chain variable region is shown in SEQ ID NO: 20.

Single-chain antibodies are various monoclonal antibodies of interest with the described biological functions. An exemplary single-chain antibody can be a single-chain antibody against CD3, such as a single-chain antibody against CD3 derived from the UCHT1 clone (US7994289B2, SEQ ID NO: 21).

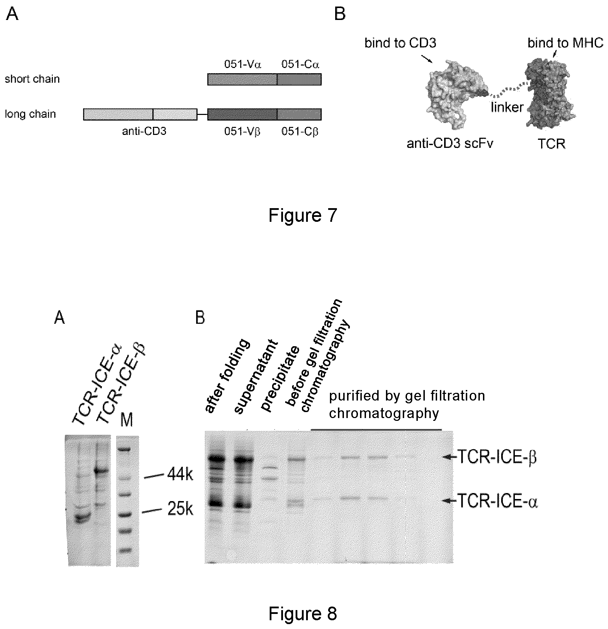

Typically, in this dual-targeting protein molecule, the α chain and β chain of the TCR molecule form a heterodimer, and the scFv is connected to the N-terminus of the variable region of the β chain of the TCR molecule. The schematic diagram of the structure of this dual-targeting protein molecule is shown in Figure 7. The scFv and the N-terminus of the variable region of the β chain can be directly connected or can be connected through a flexible peptide chain.

Herein, the flexible peptide chain can be any peptide chain without secondary structure. Suitable flexible peptide chains (linkers) are well known in the art and usually contain G and S. Exemplary flexible linkers include but are not limited to sequences containing 2-30, such as 3-20 or 3-10 amino acid residues containing G and S or consisting of G and S. The amino acid sequence of an exemplary linker is shown as SEQ ID NO: 22.

To efficiently express the modified variable regions of the α chain and β chain of the dual-targeting protein molecule of the present invention in *Escherichia coli,* the present invention uses *E.coli* codons to optimize the coding sequences of the variable regions of the α chain and β chain, and obtains the coding sequences of the variable regions of the α chain and β chain shown in SEQ ID NO: 23 and SEQ ID NO: 24.

In some embodiments, in order to obtain a stable dual-targeted protein molecule, cysteine mutations can be made at appropriate sites in the constant regions of the α and β chains, and disulfide bonds are introduced to stabilize the dimer structure. It should be noted that whether there is or is not an artificially introduced disulfide bond as described above between the constant regions, the TCR molecule of the present invention can be connected by the natural disulfide bond present in the TCR.

### Cell

The present invention also relates to host cells produced by genetic engineering using the vector or nucleic acid molecule of the present invention. The term "host cell" refers to any type of cell that can contain the nucleic acid molecule or vector of the present invention or express the TCR molecule or dual-targeted protein molecule of the present invention. In some embodiments, the characteristics of the host cell include: containing the vector of the present invention or having the nucleic acid molecule of the present invention integrated into the chromosome, and/or expressing the TCR molecule and/or dual-targeting protein molecule as described in any of the embodiments herein.

Host cells suitable for expressing the TCR of the present invention include but are not limited to prokaryotic and eukaryotic cells, such as Escherichia coli, yeast cells, insect cells, Chinese hamster ovary cells (CHO), African green monkey kidney cells (Vero cells), COS cells, HEK29 cells, etc. The host cell is preferably peripheral blood lymphocytes (PBL) or peripheral blood mononuclear cells (PBMC). More preferably, the host cell is a primary T cell.

In some embodiments, the present invention particularly relates to immune cells, especially T cells, containing the vector of the present invention or having the nucleic acid molecule of the present invention integrated into the chromosome and/or expressing the TCR molecule as described in any of the embodiments herein. T cells can be any type of T cell and can be at any stage of development, including but not limited to: CD4+/CD8+ double-positive T cells, CD4+ helper T cells (such as Th1 and Th2 cells), CD4+ T cells, CD8+ T cells (such as cytotoxic T cells), memory T cells (such as central memory T cells and effector memory T cells), naive T cells, etc. More preferably, the T cell can be derived from CD8+ T cells isolated from a patient.

In some embodiments, the cells of the present invention can also be other types of immune cells, such as natural killer cells (NK), tumor-infiltrating lymphocytes (TIL) and derived immune cells. Gene transfer to NK cells will not lead to the expression of TCR on the cell surface because NK cells do not express the CD3 molecule. However, when NK cells are induced to differentiate or are artificially constructed, the expression of the CD3 molecule will initiate the expression of the TCR molecule in NK cells.

### Pharmaceutical composition and conjugate

The present invention also provides a pharmaceutical composition comprising T cells and a pharmaceutically acceptable carrier, the T cells contain a carrier expressing the TCR molecule described in any embodiment herein or a nucleic acid molecule encoding the TCR molecule described in any embodiment of the present invention integrated in its chromosome, and/or express the TCR molecule described in any embodiment herein.

In some embodiments, the pharmaceutical composition of the present invention contains the TCR molecule or the dual-targeting protein molecule described in any embodiment herein and a pharmaceutically acceptable carrier.

Herein, pharmaceutically acceptable carriers can be selected according to specific active ingredients. For example, the pharmaceutically acceptable carrier in a pharmaceutical composition containing T cells can be various suitable carriers well known in the field and suitable for cell therapy. In a pharmaceutical composition containing the TCR molecule or dual-targeting protein molecule of the present invention, the pharmaceutically acceptable carrier can be a pharmaceutically acceptable carrier suitable for protein delivery.

Herein, the pharmaceutical composition can be administered by any appropriate route, such as parenteral, enteral, inhalation or intranasal routes. The pharmaceutical composition of the present invention can be prepared by methods well known in the field, for example, by mixing the active ingredient with a carrier or excipient under sterile conditions.

In the pharmaceutical composition of the present invention, the effective amount of active ingredients such as the T cells, TCR molecules or dual-targeting protein molecules depends on the disease or disorder to be treated, the age and condition of the individual to be treated, etc., and can be easily determined by those skilled in the art according to the actual situation. Generally speaking, the suitable dose range of the soluble TCR of the present invention can be between 25 ng/kg and 50 µg/kg.

The pharmaceutical composition of the present invention can be used for various therapeutic purposes as described below.

In some embodiments, the present invention provides a conjugate, which contains the TCR molecule described herein and a therapeutic agent or tracer covalently or otherwise bound to the TCR molecule, and is used for the treatment or diagnosis of diseases, especially tumors. The bound or conjugated therapeutic agents include but are not limited to: radionuclides, chemotherapeutic agents, antibody Fc or scFv fragments, nanoparticles, etc. Tracers for diagnostic purposes include but are not limited to: fluorescent or luminescent markers, radioactive markers, magnetic materials for MRI (magnetic resonance imaging), contrast agents for CT (computed tomography), and enzymes for detectable products.

### Use and treatment methods

The present invention also provides the use of the TCR molecule, dual-targeting protein molecule and cell (especially T cell) described in any embodiment herein in the manufacture of a medicament for treating or preventing a disease related to KRAS G12V mutant antigen in a patient, as well as the TCR molecule, dual-targeting protein molecule and cell (especially T cell) described in any embodiment herein for treating or preventing a disease related to KRAS G12V mutant antigen.

The present invention also relates to a method for treating and/or preventing a disease related to KRAS G12V mutant antigen in a patient, which includes the step of adoptive transfer of T cells containing the vector of the present invention or T cells having the nucleic acid molecule of the present invention integrated in the chromosome, and/or T cells expressing the TCR molecule described in any embodiment herein to the patient, or includes the step of administering the dual-targeting protein molecule described in any embodiment of the present invention or a pharmaceutical composition containing the dual-targeting protein molecule to the patient.

Herein, the disease related to KRAS G12V mutant antigen is tumor or cancer, which can include any one of the following: acute lymphoblastic cancer, acute myeloid leukemia, alveolar rhabdomyosarcoma, bone cancer, brain cancer, breast cancer, anal cancer, anal canal cancer or anorectal cancer, eye cancer, intrahepatic cholangiocarcinoma, joint cancer, cervical cancer, gallbladder cancer or pleural cancer, nose cancer, nasal cavity cancer or middle ear cancer, oral cancer, vaginal cancer, vulvar cancer, chronic lymphocytic leukemia, chronic myeloid cancer, colon cancer, colorectal cancer, endometrial cancer, esophageal cancer, cervical cancer, gastrointestinal carcinoid tumor, glioma, Hodgkin's lymphoma, hypopharyngeal cancer, kidney cancer, laryngeal cancer, liver cancer, lung cancer, malignant mesothelioma, melanoma, multiple myeloma, nasopharyngeal cancer, non-Hodgkin's lymphoma, oropharyngeal cancer, ovarian cancer, penile cancer, pancreatic cancer, peritoneal cancer, omental cancer and mesenteric cancer, pharyngeal cancer, prostate cancer, rectal cancer, kidney cancer, skin cancer, small intestine cancer, soft tissue cancer, stomach cancer, testicular cancer, thyroid cancer, uterine cancer, ureteral cancer, and bladder cancer. Preferred cancers are pancreatic cancer, colorectal cancer, lung cancer, endometrial cancer, ovarian cancer or prostate cancer. In some embodiments, the disease is pancreatic cancer.

Preferably, the tumor cells or cancer cells of the patient carry the KRAS G12V mutant antigen and HLA-A*11:01. Preferably, the KRAS G12V mutant antigen carried by the tumor cells or cancer cells of the patient includes but is not limited to the amino acid sequence as shown in SEQ ID NO: 16.

T cells can be isolated from patients or volunteers suffering from diseases related to KRAS G12V mutant antigen, and then they are genetically engineered in vitro to contain the vector described in any embodiment herein or have the TCR molecule described in any embodiment herein integrated in the genome and express the TCR molecule described in any embodiment herein. Subsequently, these genetically engineered cells are transfused back into the patient for treatment.

In some embodiments, the T cell is derived from the patient himself. Therefore, in these embodiments, the treatment method of the present invention further includes: (1) isolating the patient's T cell, and (2) genetically engineering the T cell in vitro to contain the vector described in any embodiment herein or have the TCR molecule described in any embodiment herein integrated in the genome and express the TCR molecule described in any embodiment herein.

The mode, timing, and dose of administration can be determined by a physician according to the age, weight, general health status of each individual patient, and the severity of the cancer being treated.

The TCR, polypeptide, protein, nucleic acid, recombinant expression vector and host cell (including its population) of the present invention can be formulated into a pharmaceutical composition in combination with another pharmaceutically active agent or medicine. Another pharmaceutically active agent or medicine can be a chemotherapeutic agent, such as asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. It can also be a monoclonal antibody-based therapeutic drug, such as targeted immune checkpoint antibody drugs (CTLA-4, PD1, PD-L1, TIGIT, LAG3, TIM3, etc.), or immune regulatory element antibody drugs (4-1BB, OX40, GITR, CD40, CD28, ICOS, CD47, etc.). In addition, it also contains other types of tumor treatment reagents, such as oncolytic viruses and vaccines (including but not limited to mRNA, DNA, protein, protein subunit, cell components or cells, etc.).

The following specific examples further illustrate the present invention. It should be understood that these examples are only used to illustrate the present invention and not to limit the scope of the present invention. The experimental methods without specific conditions in the following examples are usually carried out under conventional conditions, such as those described in Molecular Cloning-A Laboratory Manual (third edition) (2001) by Sambrook and Russel1 et al. (CSHL Press), or according to the conditions recommended by the manufacturer. Unless otherwise stated, percentages and parts are by weight. Unless otherwise stated, percentages and parts are by weight. The experimental materials and reagents used in the following examples can be obtained from commercial channels unless otherwise specified.

### Example 1: Determination of TCR gene sequence targeting KRAS G12V mutation

A functional cell population was sorted from tumor-infiltrating lymphocytes (TILs) of a tumor patient with KRAS G12V mutation (from the General Surgery Department of Jinling Hospital Affiliated to Medical School of Nanjing University). Single-cell sequencing was performed to obtain the TCR sequence. After functional verification, the TCR numbered 051 can specifically bind to the VVVGAVGVGK/HLA-A*11:01 complex. The amino acid sequence and coding sequence of its α variable region are shown as SEQ ID NO: 7 and SEQ ID NO: 11 respectively, and the amino acid sequence and coding sequence of its β variable region are shown as SEQ ID NO: 8 and SEQ ID NO: 12, respectively.
SEQ ID NO: 7 
MASAPISMLAMLFTLSGLRAQSVAQPEDQVNVAEGNPLTVKCTYSVSGNPYLFWY
VQYPNRGLQFLLKYITGDNLVKGSYGFEAEFNKSQTSFHLKKPSALVSDSALYFCAVRDI
EGAGNNRKLIWGLGTSLAVNP (The sequences of CDR1, CDR2 and CDR3 are marked in
bold and underlined in turn).
SEQ ID NO: 8

MDTWLVCWAIFSLLKAGLTEPEVTQTPSHQVTQMGQEVILRCVPISNHLYFYWYR
QILGQKVEFLVSFYNNEISEKSEIFDDQFSVERPDGSNFTLKIRSTKLEDSAMYFCASSEG
QYSYEQYFGPGTRLTVT (The sequences of CDR1, CDR2 and CDR3 are marked in bold and
underlined in turn).

### Example 2: Construction of a vector with high expression of TCR molecules

### 1. vector information

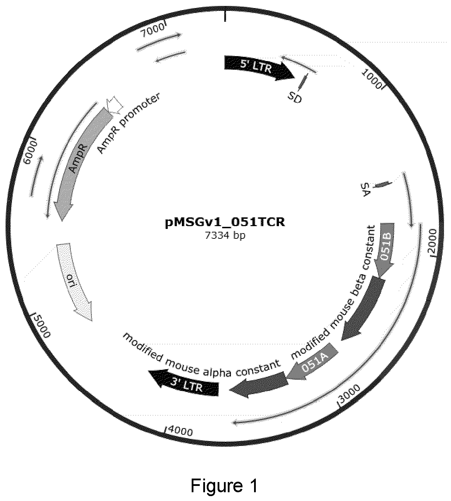

The pMSGV1 vector is used to overexpress TCR molecules in T cells. The TCR nucleic acid sequence is optimized by using human codons. The coding sequence of the α chain variable region is shown as SEQ ID NO: 11; the coding sequence of the β chain variable region is shown as SEQ ID NO: 12. In addition, the expression of TCR in T cells is completed by using a human-mouse hybrid method. The amino acid sequence of the mouse α constant region is shown as SEQ ID NO: 9, and the amino acid sequence of the β constant region is shown as SEQ ID NO: 10. The TCR α chain and β chain are connected in series by the SGSG-P2A sequence (amino acid sequence SEQ ID NO: 25, nucleic acid sequence SEQ ID NO: 26). The complete vector structure is shown in Figure 1. After the vector is constructed, it is sequenced and identified. The sequencing primers are shown as SEQ ID NO: 27, SEQ ID NO: 28, SEQ ID NO: 29, and SEQ ID NO: 30.

### 2. Plasmid extraction

After correct sequencing, NucleoBond Xtra Maxi (MACHEREY-NAGEL) is used to extract and purify the plasmid. The purified plasmid is used to measure the light absorption at 259 nm by an ultraviolet spectrophotometer to calculate the plasmid concentration. It is stored at -20°C for subsequent experiments.

### Example 3: Retroviral packaging

1. Day 1: 293T cells were digested and plated at a density of 0.6×10⁶ cells/mL. 5 mL of D10 medium (DMEM + 10% FBS) was added to a T25 flask, the cells were mixed thoroughly, and cultured overnight at 37 °C.
2. Day 2: When the confluence of 293T cells reaches about 90%, transfection was performed. The plasmid complex was prepared. The amounts of various plasmids are as follows: 3 µg of pMSGV1-051 TCR, 1.9 µg of Gag-pol, and 0.75 µg of 10A1. 300 µL of DMEM was added. 20 µL of EZ Trans cell transfection reagent (Life ilab, Shanghai) and 300 µL of DMEM were added. The PEI solution was added to the plasmid complex and vortex for 20 seconds. The mixture was added gently along the side to the 293T culture flask and the culture flask was cultured at 37°C for 16 hours. The medium was removed and preheated fresh medium was added again.
3. Day 4: After 48 hours of transfection, the supernatant was collected, filtered with a 0.45 µm filter, and aliquot and stored at -80 °C.

### Example 4: Retroviruses infect human T cells

1. PBMC cells obtained by Ficoll separation fluid (Tianjin Haoyang) were adjusted to a cell density of 2×10⁶/mL with X-VIVO (LONZA) medium containing 5% AB serum. The cells were seeded into culture plates at 1 mL/well, anti-human 50 ng/ml CD3 antibody (Takara) was added, and then 300 IU/mL interleukin-2 (Beijing Shuanglu) was added to stimulate culture for 48 hours before virus infection.
2. The following day after the activation and culture of T cells, Retronectin (Takara) with a final concentration of 15 µg/mL diluted by PBS was used to coat non-tissue treated culture plates. For 24-well plates, 300 µL was added to each well and left overnight at 4 °C for later use.
3. Two days after the activation and culture of T cells, the coated 24-well plates were taken out, the coating solution was aspirated and discarded, and the virus solution was added to the wells. 1 mL of virus solution was added to each well, and centrifuged at 32 °C and 2000 g for 2 hours.
4. The supernatant was discarded. 5×10⁵ activated T cells were added to each well of the 24-well plate, with a volume of 1 mL. The medium was T cell medium supplemented with 300 IU/ml of IL-2, and centrifuged at 32 °C and 1000 g for 10 minutes.
5. After centrifugation, the culture plates were placed in a 37 °C, 5% CO₂ incubator for culture.
6. After cell infection, T cell culture medium containing 100 IU/ml of IL-2 was added in due course to expand the cells.

### Example 5: Detection of TCR expression of T lymphocytes after infection by flow cytometry

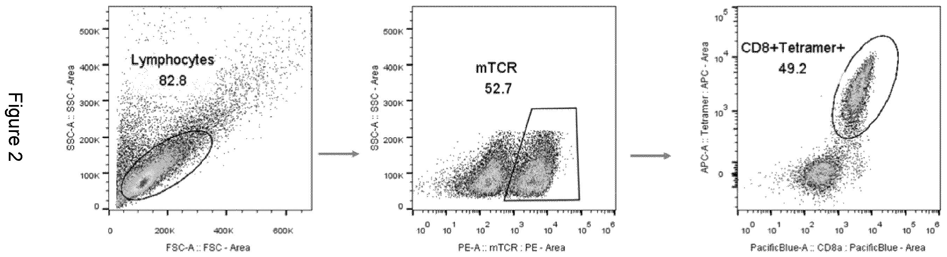

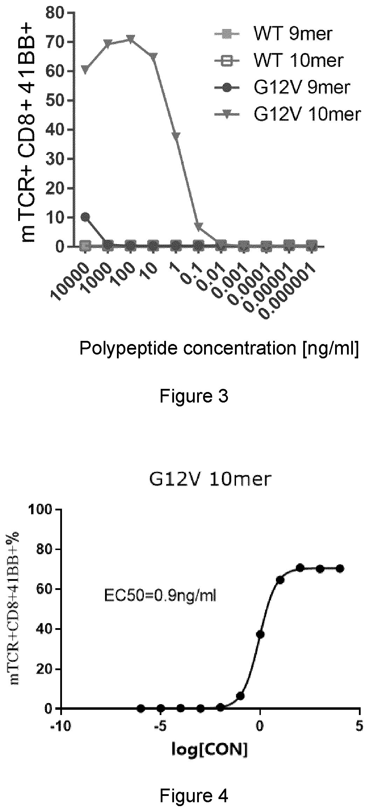

1. The TCR-T cells and NT cells (control group) were centrifuged and collected after infection, after washing once with PBS and the supernatant was discarded, the corresponding mTCR and CD8 antibodies, G12V 10mer tetramer were added, and the two group of cells were incubated for 30 minutes at 4°C in the dark. Washed with PBS, resuspended, and detected with a flow cytometer. Results are shown in Figure 2.
2. The prepared TCR-T cells were counted, and an appropriate amount of the cells were taken to centrifuge, and resuspended in X-VIVO medium containing 2% AB serum. The cell concentration was adjusted to 2×10⁶/mL, and added 100 µL per well to a 96-well plate.
3. The four peptides (sequences are shown in SEQ ID NO: 13-16) were diluted in a 10-fold gradient, with a total of 11 gradients, suspended in X-VIVO medium containing 2% AB serum, and 100 µL per well was added to the T cells, with a final concentration ranging from 10⁴ ng/mL to 10⁻⁶ ng/mL.
4. After thorough mixing, the above mixture was placed in a 5% carbon dioxide incubator and incubated at 37°C for about 16 hours.
5. mTCR+CD8+41BB+ cells were detected by flow cytometry. The results are shown in Figure 3.
6. The EC50 value of the TCR-T response to G12V 10mer was calculated. The results are shown in Figure 4, with an EC50 of 0.9 ng/ml.

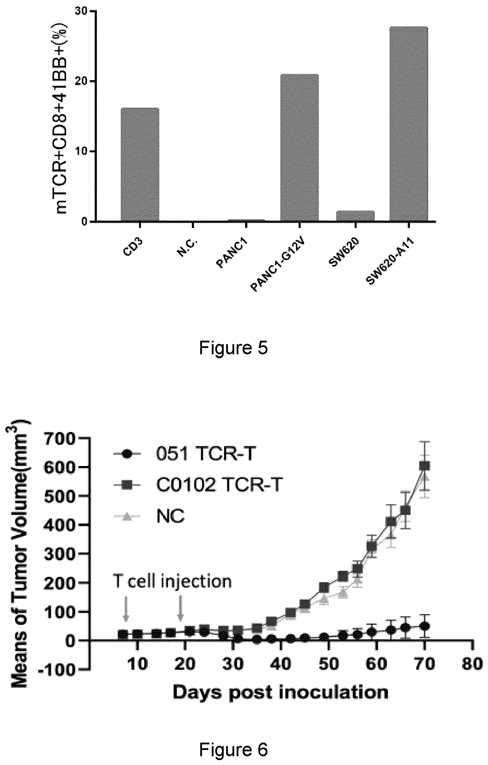

Example 6: Detection of TCR-T activation by different tumor cells.
1. The prepared TCR-T cells were counted. An appropriate amount of the cells were centrifuged, and resuspended in X-VIVO medium containing 2% AB serum, the cell concentration was adjusted to 2×10⁶/mL, then the cells were added to a 96-well plate at 100 µL per well.
2. Target cells were counted, and the concentration was adjusted to 2×10⁵ - 2×10⁶/mL, the cells were suspended in X-VIVO medium containing 2% AB serum, 100 µL T cells were added to per well, CD3 antibody was used as a positive control, blank medium was used as a negative control.
3. After thorough mixing, the above mixture was placed in a 5% carbon dioxide incubator and incubate at 37°C for about.
4. mTCR+CD8+41BB+ cells were detected by flow cytometry. The results are shown in Figure 5.

Example 7: Experiment on TCR-T inhibiting the proliferation of pancreatic cancer cells in mice.
1. 1×10⁶ PANC 1-luc-TMG cells were injected subcutaneously into each 6-8-week-old female M-NSG mouse to construct a pancreatic cancer tumor model.
2. After 7 days, the mice were divided randomly into 3 groups (6 mice in each group), namely ①TCR-T (A1101), ②TCR-T (C0102), and ③untreated group. 1×10⁷ 051 TCR-T cells were injected (recognizing HLA-A*11:01) intravenously to the mice in group ①, and 1×10⁷ TCR-T cells recognizing HLA-C*01:02 were injected to the mice in group ②. Both group ② and group ③ were used as controls. Measuring the tumor size every week and counting the tumor sizes of the three groups of mice within two months after infusion of TCR-T.

The results are shown in Figure 6. The results show that compared with the two control groups, 051 TCR-T has a significant inhibitory effect on the growth of pancreatic cancer tumors.

### Example 8: Expression, folding, and purification of TCR-ICE protein molecules

Based on recombinant DNA technology, the TCR sequence of this invention was concatenated with the anti-CD3 single-chain antibody sequence to prepare a bifunctional protein molecule that can link cytotoxic T cells and tumor cells. It is named TCR-ICE. The TCR-ICE molecule includes the TCR-α and TCR-β chain sequences from the TCR sequence of this invention (sequences see SEQ ID NO: 19, SEQ ID NO: 20), where the N-terminus of the β chain is connected to the anti-CD3 antibody sequence (sequence see SEQ ID NO: 21) (Figure 7) and is prepared through in vitro recombinant expression, folding, and purification methods.

The α and β chain sequences of the 051-TCR-ICE molecule optimized for E. coli codons are seen in SEQ ID NO: 23, SEQ ID NO: 24. After synthesizing cDNA, it was cloned into the pET21α vector. The 051-TCR-ICE-α and 051-TCR-ICE-β-αCD3 expression vectors were transformed into the E. coli strain BL21(DE3) and grown in LB medium. When the OD₆₀₀ reaches 0.8, expression was induced with a final concentration of 1 mM IPTG. After culturing at 37°C for 3 hours, bacterial inclusion bodies were collected. The inclusion bodies were washed twice with deionized water and then dissolved in a buffer solution containing 20 mM Tris-Cl (pH 8.0), 6 M guanidine hydrochloride, 0.5 mM Ethylenediaminetetraacetic acid (EDTA). After dissolution, TCR-ICE-α and TCR-ICE-β-αCD3 were mixed in equal amounts and rapidly diluted into a folding buffer (20 mM Tris-Cl (pH 8.0), 5 M urea, 0.5 mM Ethylenediaminetetraacetic acid (EDTA), 0.4 M arginine, 0.5 mM oxidized glutathione, 5 mM reduced glutathione), with a final concentration of 10 mg/ml, and folded overnight at 4°C. After centrifugation of the overnight product, the supernatant was dialyzed against a 10 mM Tris-Cl (pH 8.0) buffer for 24 hours, repeated twice. The dialysis product was purified using an anion exchange column (HiTrap Q HP, 5 ml, GE Healthcare) to purify the target protein. The product was then further purified by gel filtration chromatography (Superdex 200 10/300 GL). The purified TCR-ICE product was identified for purity by SDS-PAGE, and the SDS-PAGE results are shown in Figure 8.

### Example 9: Activation of T cells by TCR-ICE protein molecules

Typically, T cells in peripheral blood lymphocytes, when activated, exhibit high expression of 4-1BB and OX40, and simultaneously secrete gamma interferon. In this experiment, 4-1BB/OX40 and gamma interferon are used as activation markers for T cells to test the functionality of the TCR-ICE protein molecules prepared in Example 8.

The effector cells used are peripheral blood lymphocytes collected from healthy donors. The target cell lines are K562-TMG-A11 (expressing HLA-A1101 and KRAS^{G12V}), K562, CFPAC1-A11 (expressing HLA-A1101, with inherent KRAS^{G12V} mutation), CFPAC1, and other cell lines. K562-TMG-A11 and CFPAN1-A11 serve as experimental cell lines, while K562 and CFPAN1 are negative control cell lines. Reagents included in this analysis are: cell culture medium RPMI-1640, 10% FBS, D-PBS, human gamma interferon ELISA detection kit. Antibodies: 4-1BB-APC, OX40-PE, and CD3-APC-Cy7.

After culturing the effector cells and target cells at a ratio of 4:1 overnight, the expression of 4-1BB and OX40 on the surface of the effector cells is detected by flow cytometry.

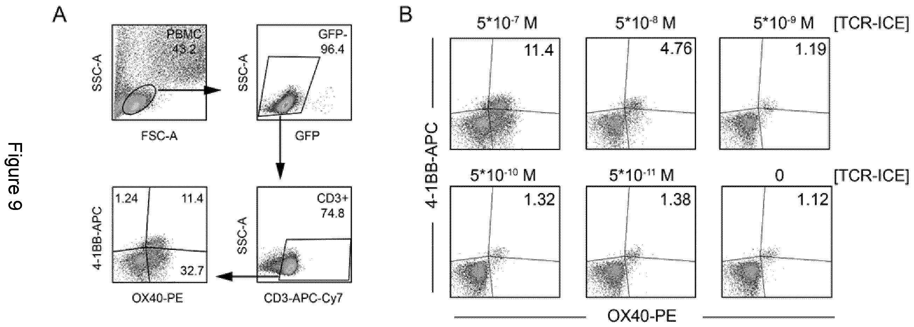

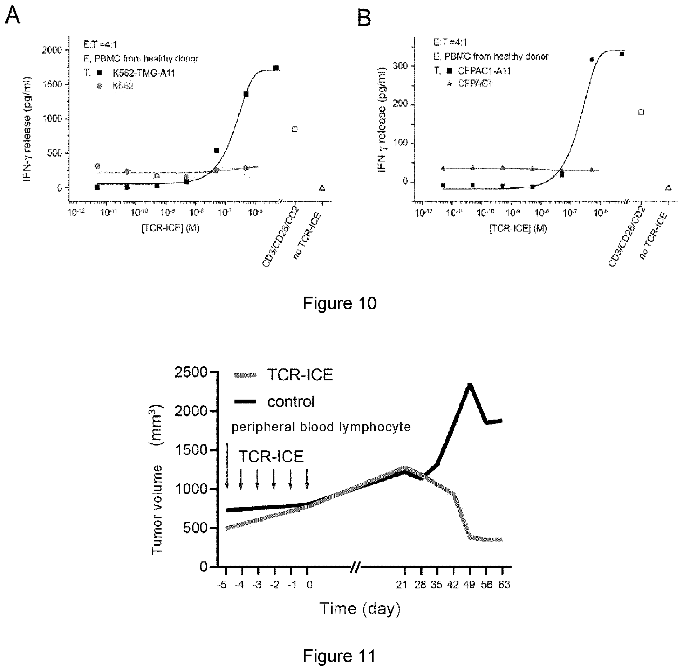

The experimental results shown in Figure 9 indicate that the TCR-ICE protein molecules can effectively activate T cells, with the activation effect showing concentration dependence. The supernatant from the co-culture of effector and target cells was tested for gamma interferon content using a human gamma interferon ELISA detection kit. The results shown in Figure 10 indicate that the target cell lines K562-TMG-A11 and CFPAN1-A11 alone cannot activate T cells; only in the presence of TCR-ICE molecules do T cells exhibit the secretion of gamma interferon. Similarly, the secretion of gamma interferon by TCR-ICE-activated T cells also shows concentration dependence. The negative control target cells K562 and CFPAN1 cannot stimulate T cells. The experimental group without the addition of TCR-ICE, after co-culturing with effector cells and K562-TMG-A11, also cannot stimulate T cells to secrete gamma interferon.

### Example 10: TCR-ICE Inhibits Tumor Growth

Study on TCR-ICE inhibiting tumor growth in mice was performed. A mouse model of pancreatic cancer with CFPAC1-luc-GFP-A11 was constructed. 1×10⁶ CFPAC1-luc-GFP-A11 cells were injected subcutaneously into two 6-8-week-old female M-NSG mice, 40 days later, peripheral blood lymphocytes from a healthy donor were injected at a dose of 5×10⁶ cells per mouse. Twenty-four hours later, TCR-ICE prepared from Example 8 was injected at a dose of 0.1 mg/kg, and PBS solution was injected as a negative control. TCR-ICE was then administered daily for the next four consecutive days, the 2^{nd} day, the 3^{rd} day, the 4^{th} day, and the 5^{th} day. The last injection of TCR-ICE was recorded as Day 0, and tumor size was recorded every seven days thereafter.

The results, as shown in Figure 11, indicate that the group injected with TCR-ICE began to show an effect in inhibiting tumor growth on Day 28. Subsequently, the tumors in the control group mice grew significantly, while the tumors in the TCR-ICE injected group gradually decreased. Around 40 days after the injection of TCR-ICE, the tumors shrank to the size before the injection, and then continued to shrink, demonstrating that TCR-ICE mediated T cell killing of tumors in the mice.

## Claims

1. A T cell receptor (TCR) molecule, wherein the TCR molecule specifically targets the KRAS G12V mutation, a CDR3 sequence of an α chain variable region of the TCR molecule comprises CAVRDIEGAGNNRKLIW (SEQ ID NO: 1) or a mutant of SEQ ID NO: 1, and/or a CDR3 sequence of a β chain variable region of the TCR molecule comprises CASSEGQYSYEQYF (SEQ ID NO: 2) or a mutant of SEQ ID NO: 2; wherein compared to SEQ ID NO: 1, the mutant of SEQ ID NO: 1 has 1-5 amino acid mutations, or has at least 80% sequence identity, and retains the binding activity of the CDR3 of the TCR α chain variable region as SEQ ID NO: 1; compared to SEQ ID NO: 2, the mutant of SEQ ID NO: 2 has 1-5 amino acid mutations, or has at least 80% sequence identity, and retains the binding activity of the CDR3 of the TCR β chain variable region as SEQ ID NO: 2;
preferably, in the TCR molecule:
a CDR1 sequence of the variable region of the α chain comprises SVSGNP (SEQ ID NO: 3) or its mutant, and a CDR2 sequence of the variable region of the α chain comprises YITGDN (SEQ ID NO: 4) or its mutant; and/or
a CDR1 sequence of the variable region of the β chain comprises SNHLY (SEQ ID NO: 5) or its mutant, and a CDR2 sequence of the variable region of the β chain comprises FYNNEI (SEQ ID NO: 6) or its mutant.

2. The TCR molecule according to claim 1, wherein:
the variable region of the α chain of the TCR molecule comprises or consists of the amino acid sequence shown in SEQ ID NO: 7, or comprises or consists of an amino acid sequence that has one or more mutations compared to the amino acid sequence shown in SEQ ID NO: 7; and/or
the variable region of the β chain of the TCR molecule comprises or consists of the amino acid sequence shown in SEQ ID NO: 8, or comprises or consists of an amino acid sequence that has one or more mutations compared to the amino acid sequence shown in SEQ ID NO: 8;
wherein the TCR molecule comprising the mutations retains the biological activity of specifically targeting the KRAS G12V mutant polypeptide by the TCR molecule comprising SEQ ID NO: 7 and SEQ ID NO: 8.

3. The TCR molecule according to claim 1, wherein:
the variable region of the α chain of the TCR molecule comprises or consists of the amino acid sequence shown as SEQ ID NO: 19, or comprises or consists of an amino acid sequence with one or more mutations compared to the amino acid sequence shown as SEQ ID NO: 19; and/or
the variable region of the β chain of the TCR molecule comprises or consists of the amino acid sequence shown as SEQ ID NO: 20, or comprises or consists of an amino acid sequence with one or more mutations compared to the amino acid sequence shown as SEQ ID NO: 20;
wherein the TCR molecule containing the mutation retains the biological activity of specifically targeting the KRAS G12V mutant polypeptide by the TCR molecule comprising SEQ ID NO: 19 and SEQ ID NO: 20.

4. The TCR molecule according to any one of claims 1-2, wherein the TCR molecule comprises a constant region of a mouse; preferably, the amino acid sequence of the α constant region of the mouse is as shown in SEQ ID NO: 9, and the amino acid sequence of the β constant region is as shown in SEQ ID NO: 10.

5. A multivalent TCR complex, wherein the multivalent TCR complex comprises two or more TCR molecules according to any one of claims 1-4.

6. A dual-targeting protein molecule capable of binding tumor cells and immune cells, wherein the dual-targeting protein molecule includes the TCR molecule targeting the KRAS G12V mutation on the surface of tumor cells according to any one of claims 1-4 and a single-chain antibody (scFv) for recruiting and redirecting immune cells to the periphery of tumor cells, wherein a signal peptide and transmembrane domain are deleted from the variable region of the α chain and the variable region of the β chain of the TCR molecule;
preferably, the amino acid sequence of the variable region of the α chain of the TCR molecule is as shown in SEQ ID NO: 19, and the amino acid sequence of the variable region of the β chain is as shown in SEQ ID NO: 20;
preferably, the single-chain antibody is an anti-CD3 single-chain antibody.

7. A nucleic acid molecule, wherein the nucleic acid molecule comprises a nucleic acid sequence encoding the TCR molecule according to any one of claims 1-4 or the dual-targeting protein molecule according to claim 6, or a complementary sequence thereof;
preferably, the nucleic acid sequence of the nucleic acid molecule is selected from: SEQ ID NO: 11, SEQ ID NO: 12, SEQ ID NO: 23 and SEQ ID NO: 24.

8. A nucleic acid construct, wherein the nucleic acid construct comprises the nucleic acid molecule according to claim 7;
preferably, the nucleic acid construct is a vector, preferably an expression vector; preferably, the vector is a viral vector or a non-viral vector;
more preferably, the vector is a retroviral vector.

9. An isolated cell, wherein the cell:
(1) comprises the nucleic acid construct according to claim 8 or comprises the nucleic acid molecule according to claim 7 integrated in the chromosome, and/or
(2) expresses the TCR molecule according to any one of claims 1-4 or the dual-targeting protein molecule according to claim 6;
preferably, the cell is an immune effector cell, preferably a T cell, NK cell or TIL cell.

10. A pharmaceutical composition, wherein the pharmaceutical composition comprises a pharmaceutically acceptable carrier and the TCR molecule according to any one of claims 1-4, the TCR complex according to claim 5, the dual-targeting protein molecule according to claim 6, the nucleic acid molecule according to in claim 7, the recombinant expression vector according to claim 8 or the cell according to claim 9.
